# EUROPÄISCHE PATENTSCHRIFT

(11) **EP 0 474 212 B1**
(45) Veröffentlichungstag und Bekanntmachung des Hinweises auf die Patenterteilung: **04.12.1996**
(21) Anmeldenummer: 91114934.2
(22) Anmeldetag: 04.09.1991
(51) Int. Cl.: C07K 14/62

(54) **Enzymatisches Verfahren zur Umwandlung von Präproinsulinen zu Insulinen**
Enzymatic process for conversion of preproinsulin to insulin
Procédé enzymatique pour la conversion de préproinsuline en insuline

(30) Priorität: 05.09.1990 DE 4028118
(43) Veröffentlichungstag der Anmeldung: 11.03.1992
(73) Patentinhaber: HOECHST AKTIENGESELLSCHAFT, 65926 Frankfurt am Main (DE)
(72) Erfinder: Dörschug, Michael, Dr., W-4630 Bochum (DE); Koller, Klaus-Peter, Dr., W-6232 Bad Soden am Taunus (DE); Marquardt, Rüdiger, Dr., W-6000 Frankfurt am Main (DE); Meiwes, Johannes, Dr., W-6238 Hofheim am Taunus (DE)

(56) Entgegenhaltungen:
- BIOCHEMICAL JOURNAL, Band 266, Nr. 3, 15 März 1990, London (GB); YAGISAWA et al., Seiten 771-775
- PROCEEDINGS OF THE 9th AMERICAN PEPTIDE SYMPOSIUM, Pierce Chemical Co., Rockford, NJ (US), 1985; C.M. DEBER et al.
- JOURNAL OF BIOLOGICAL CHEMISTRY, Band 258, Nr. 23, 10 Dezember 1983, Baltimore, MD (US); ROTH et al., Seiten 14456-14460
- PROCEEDINGS OF THE CETUS-UCLA SYMPOSIUM ON PROTEIN, 1987, Alan R. Liss Co., New York, NY (US); R. BURGESS, Seiten 435-442

## Beschreibung

Insuline bestehen aus zwei Polypeptidketten, der A-Kette, die 21 Aminosäurereste enthält und der B-Kette mit 30 Aminosäureresten. A- und B-Ketten sind über zwei Disulfidbrücken miteinander verbunden, wobei die Cysteinreste in Position A7 und B7 sowie A20 und B19 miteinander verknüpft sind. Eine dritte Disulfidbrücke besteht zwischen A6 und A11. Tierische und menschliche Insuline werden in den Pankreata in Form von Präproinsulinen gebildet. Menschliches Präproinsulin besteht z. B. aus einem Präpeptid mit 24 Aminosäureresten, daran anschließend ein Proinsulin mit 86 Aminosäureresten mit der folgenden Konfiguration: Präpeptid-B-Arg-Arg-C-Lys-Arg-A, wobei C für eine Aminosäurekette mit 31 Resten steht. Während der Exkretion aus den Langerhansschen Inseln wird das Präpeptid abgespalten und es entsteht Proinsulin. Abschließend wird die C-Kette proteolytisch gespalten und es entsteht das wirksame menschliche Insulin.

Gentechnische Methoden erlauben es im zunehmenden Maße, Präproinsuline in Mikroorganismen zu exprimieren (EP-A-347 781, EP-A-367 163). Die Abspaltung der Präprosequenzen erfolgt in der Regel chemisch und/oder enzymatisch (DE-P-3 440 988, EP-A-0264250). Bekannte enzymatische Umwandlungsmethoden beruhen auf der Spaltung mit Trypsin und Carboxypeptidase B (Kemmler W. et al. J. Biol. Chem., 246 (1971) 6786-6791; EP-A-195 691; EP-B-89007). Nachteil dieser Methoden ist die Entstehung von großen Mengen an Nebenprodukten, die nur schwer aus der Reaktionslösung abzutrennen sind. Besonders bei der Umwandlung von menschlichem Präproinsulin in menschliches Insulin (Humaninsulin, HI) entstehen größere Mengen von Des-Thr(B30)-Humaninsulin (Des-Thr(B30)-HI). Dieses Nebenprodukt unterscheidet sich von HI nur durch das Fehlen einer endständigen Aminosäure und ist sehr schwer aus den Reaktionslösungen abtrennbar.

Zur Verminderung dieser Nebenproduktbildung können bestimmte Schwermetalle, insbesondere Nickel, dem Spaltungsansatz zugegeben werden (EP-A 0264 250). Eine derartige Reaktionsführung ist unter großtechnischen Gesichtspunkten aufgrund der hohen Abwasserbelastungen mit Schwermetallen nicht wünschenswert. Es besteht daher Bedarf an einer möglichst spezifischen und umweltverträglichen Umwandlung von Präproinsulinen.

Clostripain (Clostriopeptidase B; EC 3.4.22.8) ist ein Enzym aus dem Kulturfiltrat von Clostridium histolyticum mit einem Molekulargewicht von etwa 30 000 bis 80 000, das sowohl proteolytische als auch Amidase-Esterase Aktivität aufweist (Mitchell, W. M, Harrington, W. F., J. of Biol. Chem., 243 (18), 4683 - 4692, 1968). Sie zeichnet sich durch eine hohe Spezifität für Arg-C-Bindungen aus. So wird in der isolierten B-Kette des Insulins die Arg-Gly Bindung durch Clostripain 500-fach schneller gespalten als die Lys-Ala Bindung und im Glucagon werden nur die Arg-Arg, die Arg-Ala und die Lys-Tyr gespalten. Die Hydrolysegeschwindigkeit für diese Bindungen stehen im Verhältnis 1, 1/7 und 1/300 zueinander. (Labouesse, B., Bull. Soc. Chim. Biol., 42, 1293, 1960). Überraschenderweise wurde nun gefunden, daß Clostripain Präproinsulin spezifisch C-terminal hinter Arginin spaltet, ohne daß hinter dem in der B-Kette vorhandenen Arginin (B22) eine nennenswerte Spaltung der Aminosäurekette auftritt.

Die Erfindung betrifft somit ein Verfahren zur Hydrolyse der Aminosäurekette von Präproinsulin der Formel I, 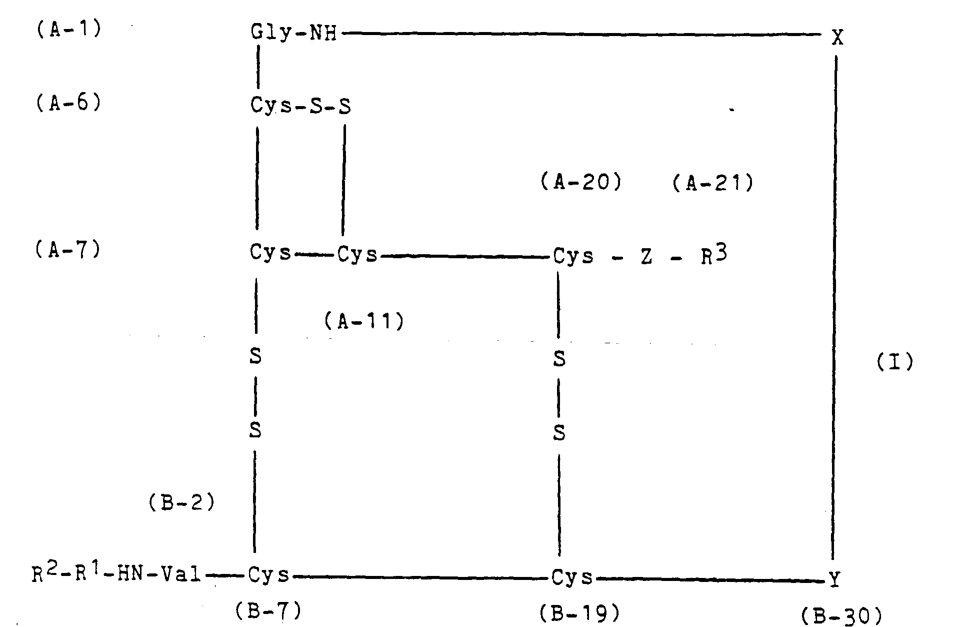 in welcher
- R¹: für n Aminosäuren steht, wobei n die ganze Zahl 0 oder 1 bedeutet,
- R²: für Wasserstoff,
eine chemisch oder enzymatisch abspaltbare Aminosäure oder ein Peptid mit 2 bis 30 Aminosäureresten,
- R³: für eine Hydroxygruppe,
eine Aminosäure oder
ein Peptid mit 2 bis 10 Aminosäuren,
- X: für L-Arginin oder
ein Peptid mit 2 bis 45 Aminosäuren, an dem C-terminal
und N-terminal ein L-Argininrest steht,
- Y: für eine genetisch kodierbare Aminosäure,
- Z: für eine genetisch kodierbare Aminosäure,
A1 bis A20 und B2 bis B29 für eine natürliche oder durch Austausch einer oder mehrerer Aminosäurereste veränderte Aminosäuresequenz menschlicher oder tierischer Insuline steht,
das dadurch gekennzeichnet ist, daß das Präproinsulin in Gegenwart von Clostripain hydrolysiert und gegebenenfalls mit Carboxypeptidase B in das entsprechende Insulin überführt wird.

Die Aminosäuresequenz von Peptiden und Proteinen wird vom N-terminalen Ende der Aminosäurekette an bezeichnet. Proteasen hydrolysieren die Peptidbindung zwischen den Aminosäuren von Peptiden und Proteinen. Clostripain hydrolysiert L-Arginin enthaltende Peptide oder Proteine spezifisch hinter Arginin. Als Reaktionsprodukte der Präproinsulinhydrolyse entstehen Insulinderivate oder Polypeptide, die C-terminal einen Argininrest besitzen, oder Aminosäuren.

Unter dem Begriff natürliche Aminosäuren werden beispielsweise Gly, Ala, Ser, Thr, Val, Leu, Ile, Asp, Asn, Glu, Gln, Cys, Met, Tyr, Phe, Pro, Hyp, Trp, Arg, Lys Hyl, Orn, Cit oder His verstanden.

Für den Begriff genetisch kodierbare Aminosäure stehen z. B. Gly, Ala, Ser, Thr, Val, Leu, Ile, Asp, Asn, Glu, Gln, Cys, Met, Arg, Lys, His, Tyr, Phe, Trp, Pro oder Selenocystein.

Bevorzugt sind die Präproinsuline der Formel I,
in welcher
- R¹: für Phe,
- R²: für Wasserstoff, eine natürliche Aminosäure oder ein Peptid, mit 2 bis 30 natürlichen Aminosäuren, das C-terminal mit L-Arginin endet,
- R³: für eine Hydroxygruppe,
eine natürliche Aminosäure
oder ein Peptid, mit 2 bis 10 natürlichen Aminosäuren,
- X: für L-Arginin oder
eine C-Kette eines menschlichen oder tierischen Proinsulins,
- Y: für eine Aminosäure aus der Gruppe Thr, Ala oder Ser,
- Z: für eine Aminosäure aus der Gruppe Asn, Gln, Asp, Glu, Gly, Ser, Thr, Ala oder Met,
A1 bis A20 und B2 bis B29 für die Aminosäuresequenz menschlicher oder tierischer Insuline steht.

Besonders bevorzugt sind Präproinsuline der Formel I,
in welcher
- R¹: für Phe,
- R²: für Wasserstoff
oder ein Peptid, mit 2 bis 30 natürlichen Aminosäuren, das C-terminal mit L-Arginin endet,
- R³: für eine Hydroxygruppe,
eine natürliche Aminosäure
oder ein Peptid, mit 2 bis 10 natürlichen Aminosäuren,
- X: für L-Arginin oder
eine C-Kette von Human-, Schweine- oder Rinder-Proinsulin,
- Y: für Thr,
- Z: für Asn
A1 bis A20 und B2 bis B29 für die Aminosäuresequenz von Human-, Schweine- oder Rinder-Insulin steht.

Insbesondere bevorzugt sind Insuline beispielsweise InsuArg mit folgender Aminosäuresequenz:

NH2-Asp Thr Thr Val Ser Glu Pro Asp Pro Asn Ser Asn Gly Arg
Phe Val Asn Gln His Leu Cys Gly Ser His Leu Val Glu Ala Leu
Tyr Leu Val Cys Gly Glu Arg Gly Phe Phe Tyr Thr Pro Lys Thr
Arg Gly Ile Val Glu Gln Cys Cys Thr Ser Ile Cys Ser Leu Tyr
Gln Leu Glu Asn Tyr Cys Asn-COOH

Clostripain (EC 3.4.22.8.) ist eine extracelluläre Thiolprotease aus Clostridien. Das Enzym ist ein Heterodimer und besitzt keinerlei Homologie mit anderen bekannten Thiolproteasen. Das Enzym besitzt eine außergewöhnlich hohe Spezifität für Arg-XXX Bindungen insbesondere Arg-Pro. Sie läßt sich charakterisieren durch ein Molekulargewicht zwischen 30 000 und 80 000 und einen isoelektrischen Punkt, von pH 4.8 bis 4.9. Als Aktivatoren wirken z. B. Cystein, Mercaptoethanol, Dithiothreitol oder Calciumionen.

In Gegenwart von z. B. Tosyl-L-Lysin-chlormethylketon, Wasserstoffperoxid, EDTA, Co²⁺-, Cu²⁺-, Cd²⁺-Ionen oder Citrat wird Clostripain gehemmt.

Die Herstellung von Clostripain erfolgt mit Hilfe von Mikroorganismen durch Fermentation. Bei diesen Verfahren werden Clostridien kultiviert, bis sich Clostripain in dem Nährmedium anhäuft. Geeignet ist beispielsweise Clostridium histolyticum, insbesondere Clostridium histolyticum DSM 627. Auch Mutanten und Varianten der genannten Mikroorganismen sind geeignet, sofern sie Clostripain synthetisieren.

Die Anzucht erfolgt anaerob einzeln oder in Mischkultur, beispielsweise submers in stehender Kultur in Abwesenheit von Sauerstoff oder in Fermentern, gegebenenfalls unter Einführung von Stickstoff, Edelgasen oder anderen Gasen, außer Sauerstoff. Die Fermentation erfolgt in einem Temperaturbereich von etwa 10 bis 45°C, vorzugsweise etwa 25 bis 40°C, insbesondere 30 bis 38°C. Es wird in einem pH-Bereich zwischen 5 und 8,5, vorzugsweise zwischen 5,5 und 8 fermentiert. Unter diesen Bedingungen zeigt die Kulturbrühe im allgemeinen nach 1 bis 3 Tagen eine nennenswerte Akkumulation des Enzyms. Die Synthese des Clostripains beginnt in der späten 10g-Phase und erreicht ihr Maximum in der stationären Wachstumsphase. Die Produktion des Enzyms kann mit Hilfe von Aktivitätstests verfolgt werden (Mitchell W., Meth. of Enzym., Bd. 47 (1977), Seite 165-170).

Die zur Produktion des Clostripains verwendete Nährlösung enthält 0,2 bis 6 %, bevorzugt 0,5 bis 3 %, organische Stickstoffverbindungen sowie anorganische Salze. Als organische Stickstoffverbindungen kommen in Betracht: Aminosäuren, Peptone, ferner Fleischextrakte, gemahlene Samen, beispielsweise von Mais, Weizen, Bohnen, Soja oder der Baumwollpflanze, Destillationsrückstände der Alkoholherstellung, Fleischmehle oder Hefeextrakte. An anorganischen Salzen kann die Nährlösung beispielsweise Chloride, Carbonate, Sulfate oder Phosphate der Alkali- oder Erdalkalimetalle, Eisen, Zink und Mangan enthalten, aber auch Ammoniumsalze und Nitrate.

Die optimalen Fermentations-Bedingungen sind für jeden Mikroorganismus zwar unterschiedlich, aber entweder dem Fachmann schon bekannt oder aber in leichten Vorversuchen festzustellen. Die Reinigung von Clostripain kann nach klassischen Verfahren z.B. über Ammoniumsulfat-Fällung, Ionenaustauscher- oder Gelpermeationschromatographie erfolgen. Die Koppelung des Enzyms ist nach gängigen Methoden möglich (Colowick und Kaplan, Meth. Enzymol., Vol. XLIV).

Für die enzymatische Umsetzung können sowohl ganze Zellen in freier oder immobilisierter Form als auch das isolierte Enzymprodukt, das ebenfalls trägergebunden sein kann, eingesetzt werden.

Die Spaltung der Präproinsuline der Formel I mit Clostripain erfolgt in einem wässrigen Medium, dem auch wassermischbare organische Bestandteile, wie z.B. Alkohole, Ketone, Harnstoff, N,N-Dimethylformamid zugesetzt werden können. Insbesondere können dem Reaktionsansatz zur besseren pH-Wert-Kontrolle der Reaktion entsprechende anorganische oder organische Puffer wie Phosphat, Tris, Glycin, HEPES u.ä. zugesetzt werden. Die Konzentration der Präproinsuline während der Spaltung liegt beispielsweise zwischen 0,01 mg/ml und 100 mg/ml, bevorzugt zwischen 0,1 mg/ml und 10 mg/ml. Das Verhältnis von Präproinsulin zu Clostripain beträgt (mg zu Units (U)) 1:0,01 bis 1:1000, bevorzugt 1:0,1 bis 1:50.

Die Temperatur bei der Reaktion ist ebenfalls in einem weiten Rahmen variierbar. Bevorzugt ist ein Temperaturbereich zwischen 0°C und +80°C, besonders bevorzugt eine Temperatur zwischen +20°C und +40°C.

Der pH-Wert der Reaktion kann zwischen pH 4 und pH 12 variieren, besonders bevorzugt ist der Bereich zwischen pH 6 und pH 9.

Die Zeit, die für die Umwandlung der Präproinsuline in die entsprechenden Intermediate benötigt wird, kann je nach Reaktionsbedingungen in einem weitem Rahmen variiert werden z.B. kann sie zwischen 15 min und 48 h liegen, bevorzugt ist eine Reaktionsdauer zwischen 1 h und 6 h.

Das Enzym wird vor dem Einsatz in geeigneter Weise in Gegenwart eines Mercaptans aktiviert. Als Mercaptane kommen dabei im Prinzip alle Verbindungen in Frage, die SH-Gruppen enthalten, bevorzugt wird DTT, DTE, Mercaptoethanol, Thioglycolsäure oder Cystein verwendet. Die Konzentration des Mercaptans kann in einem breiten Rahmen variiert werden, bevorzugt sind Konzentrationen zwischen 0.1 mM und 100 mM. Des weiteren enthält der Aktivierungspuffer Ca²⁺-Ionen, vorzugsweise CaCl₂. Die Aktivierung findet statt zwischen pH 4 und pH 12, vorzugsweise zwischen pH 6 und pH 8, besonders bevorzugt ist der Bereich pH 7 bis pH 8. Zur Aufrechterhaltung des pH-Wertes kann eine geeignete Puffersubstanz, z. B. Tris, HEPES, Glycin u. ä. zugesetzt werden. Die Aktivierungstemperatur kann zwischen 0°C und 60°C liegen, bevorzugt ist der Bereich 0°C bis 10°C, besonders bevorzugt 0°C bis 5°C. Das so aktivierte Enzym kann entweder direkt verwendet werden, oder ggf. durch Chromatographie über ®Ultrogel AcA 202 vom Aktivierungspuffer befreit werden.

Die erfindungsgemäße Spaltung von Präproinsulin der Formel I führt zu Insulinderivaten mit Argininresten am C-terminalen Ende der Insuline und den entsprechenden abgespaltenen Aminosäuren und/oder Peptiden. Die Insulinderivate können gewünschtenfalls mit Carboxypeptidase B in die entsprechenden Insuline überführt werden. Dies kann im gleichen Reaktionsansatz gleichzeitig mit Clostripain zusammen geschehen aber auch unter den obengenannten Reaktionsbedingungen hintereinander, wobei das Insulinderivat gegebenenfalls vor der Carboxypeptidase B-Behandlung mit an sich bekannten Methoden wie z.B. Chromatographie oder Kristallisation isoliert werden kann. Carboxypeptidase B kann in gelöster oder in immobilisierter Form eingesetzt werden. Das Verhältnis von Carboxypeptidase B zu Insulinderivat beträgt (Gewicht zu Gewicht) etwa 1:10 bis 1:5000, bevorzugt etwa 1:500 bis 1:3500 und besonders bevorzugt etwa 1:1000 bis 1:3000.

Das Verhältnis von Carboxypeptidase B zu Clostripain beträgt (Gewicht zu Gewicht) etwa 1:1 bis 10:1 und bevorzugt 2:1 bis 5:1.

Die Reaktionsprodukte der Clostripain- und/oder Carboxypeptidase B-Spaltung können beispielsweise ausgefällt werden durch Erniedrigung des pH-Wertes und/oder mit Hilfe bekannter säulenchromatographischer Methoden gereinigt werden. Das erhaltene Insulin kann in üblichen Darreichungsformen zubereitet und als Arzneimittel zur Behandlung des Diabetes mellitus verwendet werden.

In den folgenden Beispielen wird das erfindungsgemäße Verfahren im einzelnen beschrieben. Prozentangaben beziehen sich auf das Gewicht wenn nicht anders angegeben.

### Beispiel 1

Die Kultivierung von Clostridium histolyticum DSM 627 erfolgt in einer Nährlösung folgender Zusammensetzung:

| | |
|---|---|
| Caseinpepton | 3 % |
| Fleischextrakt | 3 % |
| Hefeextrakt | 0,5 % |
| Cystein | 0,05 % |
| KH₂PO₄ | 0,15 % |
| pH 7,2 | |

Die Vorkultur wird 1 % angeimpft. Die Kultivierung erfolgt in geschlossenen Flaschen unter anaeroben Bedingungen bei 37°C für etwa 2 Tage. Die Stammhaltung der Mikroorganismen erfolgt in obengenannter Nährlösung mit 50 % Glycerin bei -20°C. Der Fermenter wird 1 % mit der Vorkultur angeimpft. Es wird ein Fermenter mit 10 1 Inhalt und 8 1 Nährlösung angeimpft. Die Kultivierung erfolgt unter Stickstoffbegasung bei 33°C, 24 h, und konstanter pH von 7,0. Es wurde eine Enzymaktivität von 20 000 U/l im Kulturfiltrat gemessen (Mitchell W., Meth. of Enzym., Bd. 47, S. 165-170, 1977).

Die Aufarbeitung erfolgte durch Abzentrifugation der Zellen bei etwa 6000 g, Sterilfiltration durch einen Filter mit 0,22 µm Porengröße, Zugabe von 60 % eiskaltem (-20°C) Methanol zum Filtrat. Dann wurde die Lösung 24 h bei -20°C gehalten und anschließend abzentrifugiert (8000 g). Das Pellet wurde in sterilem Aqua bidest. gelöst und abzentrifugiert (12 000 g). Im Pellet wurde eine Enzymaktivität von 300 U/ml, 200 U/mg Protein, gemessen. Die Ausbeute betrug 75 % der gemessenen Aktivität im Fermenter.

### Beispiel 2

Die Anzucht der Zellen erfolgte wie in Beispiel 1. Das Produktionsmedium im Fermenter bestand aus:

| | |
|---|---|
| Protease Pepton (Difco) | 5 % |
| Cystein | 0,05 % |
| KH₂ PO₄ | 0,15 % |
| pH 7,2 | |

und wurde 2 % aus einer Vorkultur angeimpft. Clostripainaktivität war 45 000 U/ml im Kulturfiltrat.

Die Aufarbeitung erfolgte durch Tangential-Flow-Filtration an 0,3 µm Membranen (Filtron, Omega Membran) zur Abtrennung der Zellen und Tangential-Flow-Filtration an 10 KD Membranen (Filtron, Omega Membran) zur Aufkonzentrierung des gelösten Clostripains. Der Aufkonzentrierungsfaktor beträgt 20. Anschließend wurde das Konzentrat entsalzt und über DEAE-Cellulose chromatographiert. Clostripainaktivität 1000 U/ml; Ausbeute 85 %. Die Lagerung bis zum Einsatz des Enzympräparats erfolgt bei -20°C.

### Beispiel 3

### A. Aktivierung von Clostripain

50 µl Enzympräparation (200 U/ml, 286 U/mg aus Beispiel 1)
1 µl Aktivierungspuffer (250 mM DTT, 125 mM CaCl₂)

- Gehalt im Ansatz:

| | |
|---|---|
| DTT | 5 mM |
| CaCl₂ | 2,5 mM |

- Inkubation 2 h auf Eis
- für die Spaltungsreaktion wird das Enzym 1:40 mit 25 mM Tris/HCl Puffer pH 7,8 verdünnt.

### B. Clostripain-Spaltungsansatz zur Freisetzung von (B31)Arg-Insulin

100 µl Insu-Arg (1 mg/ml)
20 µl KCl (1 M)
5 µl Tris/HCl (1 M, pH 7,8)
55 µl H₂O
20 µl Clostripain (1:40 Verdünnung)

- Gehalt im Ansatz:

| | |
|---|---|
| Insu-Arg | 0,5 mg/ml |
| Clostripain | 2,5 U/ml |
| DTT | 12,5 µM |
| Tris/HCl | 25 mM |
| KCl | 100 mM |
| CaCl₂ | 6 µM |

- Inkubation 1 - 2 h bei 28°C, die Reaktion kann leicht über HPLC kontrolliert werden, danach Abstoppen der Reaktion mit Tosyl-L-Lysin-chlormethyl Keton (TLCK).
- Abstoppen der Reaktion durch Zugabe von 1 µl TLCK (15 mM)
- Lagerung bei 4°C
- Ergebnis: Human-Insulin-Arg. Mit der HPLC ist keine Human-Insulin(DesB30)-Bildung meßbar.

### C. Carboxypeptidase B - Spaltungsansatz zur Freisetzung von Humaninsulin

200 µl Clostripain-Spaltungsansatz
10 µl Carboxypeptidase B (1:100 Verdünnung)

- Carboxypeptidase-Gehalt im Ansatz: 2,5 µg/ml
- Inkubation 2 - 4 h bei 280C, die Reaktion ist durch HPLC leicht zu überprüfen
- für die Reaktion wird Carboxypeptidase B (759 U/ml, 150 U/mg, aus Schweinepankreas) 1:1000 mit 25 mM Tris/HCl Puffer pH 7,8 verdünnt
- Ergebnis: Human-Insulin. Keine Insulin(DesB30)-Bildung, die mit der HPLC meßbar ist.

### D. HPLC-Analytik

Die Spaltungen wurden kontrolliert unter Verwendung einer RP 18 Säule (0,125 M NH₄ (SO₄)₂, mit H₂SO₄ auf pH 4 eingestellt, 25-50 % Acetonitril-Gradient) bzw. einer C 8 Säule (0,1 % TFA, 20 % - 50 % Acetonitril-Gradient).

### Beispiel 4

- Clostripain:: 200 U/ml (aus Beispiel 1)
- Aktivierungspuffer:: 500 mM Tris/HCl, pH 7,8
100 mM DTT
25 mM CaCl₂
- Aktivierung:: 100 µl Clostripainlösung
10 µl Aktivierungspuffer
- Faltungsansatz:: 35,7 mg menschlisches Prä-B-Kette-A-Ketteninsulin-S-Sulfonat
315 µl 1 M Mercaptoethanol
105 µl 1 M Ascorbinsäure
100 ml 20 mM Glycinpuffer, pH 10.7

Die Faltung erfolgte über Nacht im Kühlraum bei 4°C , die Faltungsausbeute betrug 0,152 mg/ml. Nach Abtrennung von Verunreinigungen durch pH-Fällung bei pH 5,0 wird Tris in einer Endkonzentration von 50 mM zugegeben und der pH-Wert auf 7,8 mit HCl eingestellt. Es wurden 30 µl Enzymlösung zugegeben. Die Spaltung erfolgte bei 30°C und wurde mittels HPLC verfolgt.
- Ergebnis:: Das obengenannte Präproinsulin läßt sich zu menschlichem InsulinArg spalten. Die Insulin(DesB30)-Bildung ist minimal.

## Patentansprüche

1. Verfahren zur Hydrolyse der Aminosäurekette von Präproinsulin der Formel I, 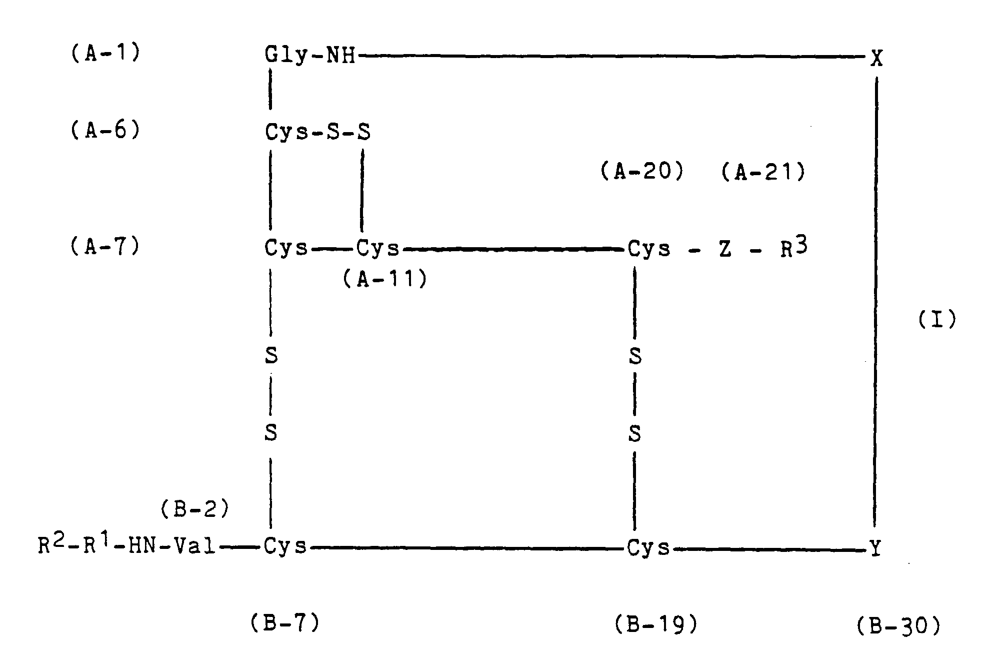 in welcher
R¹ für n Aminosäuren steht, wobei n die ganze Zahl 0 oder 1 bedeutet,
R² für Wasserstoff,
eine chemisch oder enzymatisch abspaltbare Aminosäure oder ein Peptid mit 2 bis 30 Aminosäureresten,
R³ für eine Hydroxygruppe,
eine Aminosäure oder
ein Peptid mit 2 bis 10 Aminosäuren,
X für L-Arginin oder
ein Peptid mit 2 bis 45 Aminosäuren, an dem C-terminal und N-terminal ein L-Argininrest steht,
Y für eine genetisch kodierbare Aminosäure,
Z für eine genetisch kodierbare Aminosäure,
A1 bis A20 und B2 bis B29 für eine natürliche oder durch Austausch einer oder mehrerer Aminosäurereste veränderte Aminosäuresequenz menschlicher oder tierischer Insuline steht,
dadurch gekennzeichnet, daß das Präproinsulin in Gegenwart von Clostripain hydrolysiert und gegebenenfalls mit Carboxypeptidase B in das entsprechende Insulin überführt wird.

2. Verfahren gemäß Anspruch 1, dadurch gekennzeichnet, daß das Präproinsulin der Formel I,
in welcher
R¹ für Phe,
R² für Wasserstoff, eine natürliche Aminosäure
oder ein Peptid, mit 2 bis 30 natürlichen Aminosäuren, das C-terminal mit L-Arginin endet,
R³ für eine Hydroxygruppe,
eine natürliche Aminosäure
oder ein Peptid, mit 2 bis 10 natürlichen Aminosäuren,
X für L-Arginin oder
eine C-Kette eines menschlichen oder tierischen Proinsulins,
Y für eine Aminosäure aus der Gruppe Thr, Ala oder Ser,
Z für eine Aminosäure aus der Gruppe Asn, Gln, Asp, Glu, Gly, Ser, Thr, Ala oder Met,
Al bis A20 und B2 bis B29 für die Aminosäuresequenz menschlicher oder tierischer Insuline steht, eingesetzt wird.

3. Verfahren gemäß Anspruch 1 oder 2, dadurch gekennzeichnet, daß das Präproinsulin der Formel I,
in welcher
R¹ für Phe,
R² für Wasserstoff
oder ein Peptid, mit 2 bis 30 natürlichen Aminosäuren, das C-terminal mit L-Arginin endet,
R³ für eine Hydroxygruppe,
eine natürliche Aminosäure
oder ein Peptid, mit 2 bis 10 natürlichen Aminosäuren,
X für L-Arginin oder
eine C-Kette von Human-, Schweine- oder Rinder-Proinsulin;
Y für Thr,
Z für Asn
A1 bis A20 und B2 bis B29 für die Aminosäuresequenz von Human-, Schweine- oder Rinder-Insulin steht, eingesetzt wird.

4. Verfahren nach einem oder mehreren der Ansprüche 1 bis 3, dadurch gekennzeichnet, daß bei einem pH-Wert zwischen 4 und 12, vorzugsweise zwischen 6 und 9, gearbeitet wird.

5. Verfahren nach einem oder mehreren der Ansprüche 1 bis 4, dadurch gekennzeichnet, daß die Konzentration der Präproinsuline der Formel I zwischen 0,01 mg/ml und 100 mg/ml, vorzugsweise zwischen 0,1 mg/ml und 10 mg/ml, liegt.

6. Verfahren nach einem oder mehreren der Ansprüche 1 bis 5, dadurch gekennzeichnet, daß das Präproinsulin-Clostripain-Verhältnis (mg zu Units) 1:0,01 bis 1:1000, vorzugsweise 1:0,1 bis 1:50, beträgt.

7. Verfahren nach einem oder mehreren der Ansprüche 1 bis 6, dadurch gekennzeichnet, daß die Reaktionstemperatur zwischen 0°C und +80°C, vorzugsweise zwischen +20°C und +40°C, liegt.

8. Verfahren nach einem oder mehreren der Ansprüche 1 bis 7, dadurch gekennzeichnet, daß gleichzeitig Carboxypeptidase B anwesend ist oder nach Spaltung mit Clostripain eingesetzt wird.

9. Verfahren nach einem oder mehreren der Ansprüche 1 bis 8, dadurch gekennzeichnet, daß Clostripain und/oder Carboxypeptidase B in immobilisierter Form vorliegen.

## Claims

1. A process for the hydrolysis of the amino-acid chain of preproinsulin of the formula I 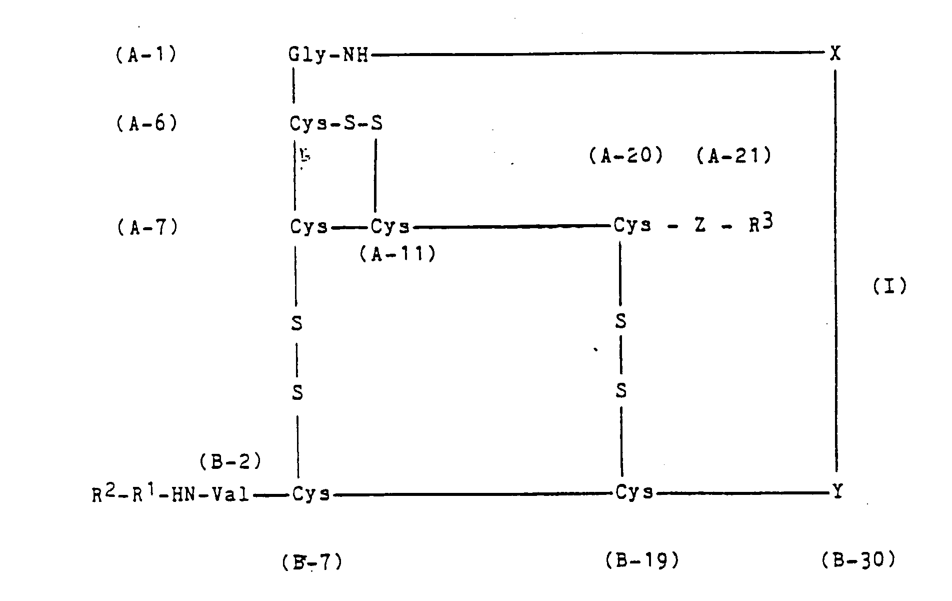 in which
R¹ is n amino acids, where n is the integer 0 or 1,
R² is hydrogen, an amino acid which can be cleaved off chemically or enzymatically, or a peptide with 2 to 30 amino-acid residues,
R³ is a hydroxyl group, an amino acid or a peptide with 2 to 10 amino acids,
X is L-arginine or a peptide with 2 to 45 amino acids and with a C-terminal and N-terminal L-arginine residue,
Y is a genetically encodable amino acid,
Z is a genetically encodable amino acid,
A1 to A20 and B2 to B29 is an amino-acid sequence, which is natural or has been modified by replacement of one or more amino-acid residues, of human or animal insulins, which comprises the preproinsulin being hydrolyzed in the presence of clostripain and, where appropriate, being converted with carboxypeptidase B into the corresponding insulin.

2. The process as claimed in claim 1, in which is used the preproinsulin of the formula I in which
R¹ is Phe,
R² is hydrogen, a natural amino acid or a peptide with 2 to 30 natural amino acids and with C-terminal L-arginine at the end,
R³ is a hydroxyl group, a natural amino acid or a peptide with 2 to 10 natural amino acids,
X is L-arginine or a C chain of a human or animal proinsulin,
Y is an amino acid from the group comprising Thr, Ala or Ser,
Z is an amino acid from the group comprising Asn, Gln, Asp, Glu, Gly, Ser, Thr, Ala or Met,
A1 to A20 and B2 to B29 are the amino-acid sequence of human or animal insulins.

3. The process as claimed in claim 1 or 2, in which is used the preproinsulin of the formula I in which
R¹ is Phe,
R² is hydrogen or a peptide with 2 to 30 natural amino acids and with C-terminal L-arginine at the end,
R³ is a hydroxyl group, a natural amino acid or a peptide with 2 to 10 natural amino acids,
X is L-arginine or a C chain of human, porcine or bovine proinsulin,
Y is Thr,
Z is Asn
A1 to A20 and B2 to B29 are the amino-acid sequence of human, porcine or bovine insulin.

4. The process as claimed in one or more of claims 1 to 3, which is carried out at a pH between 4 and 12, preferably between 6 and 9.

5. The process as claimed in one or more of claims 1 to 4, wherein the concentration of the preproinsulins of the formula I is between 0.01 mg/ml and 100 mg/ml, preferably between 0.1 mg/ml and 10 mg/ml.

6. The process as claimed in one or more of claims 1 to 5, wherein the preproinsulin/clostripain ratio (mg to units) is 1:0.01 to 1:1,000, preferably 1:0.1 to 1:50. ·

7. The process as claimed in one or more of claims 1 to 6, wherein the reaction temperature is between 0°C and +80°, preferably between +20°C and +40°C.

8. The process as claimed in one or more of claims 1 to 7, wherein carboxypeptidase B is present at the same time or is employed after cleavage with clostripain.

9. The process as claimed in one or more of claims 1 to 8, wherein clostripain and/or carboxypeptidase B are present in immobilized form.

## Revendications

1. Procédé d'hydrolyse de la chaîne des acides aminés de préproinsuline de Formule I, 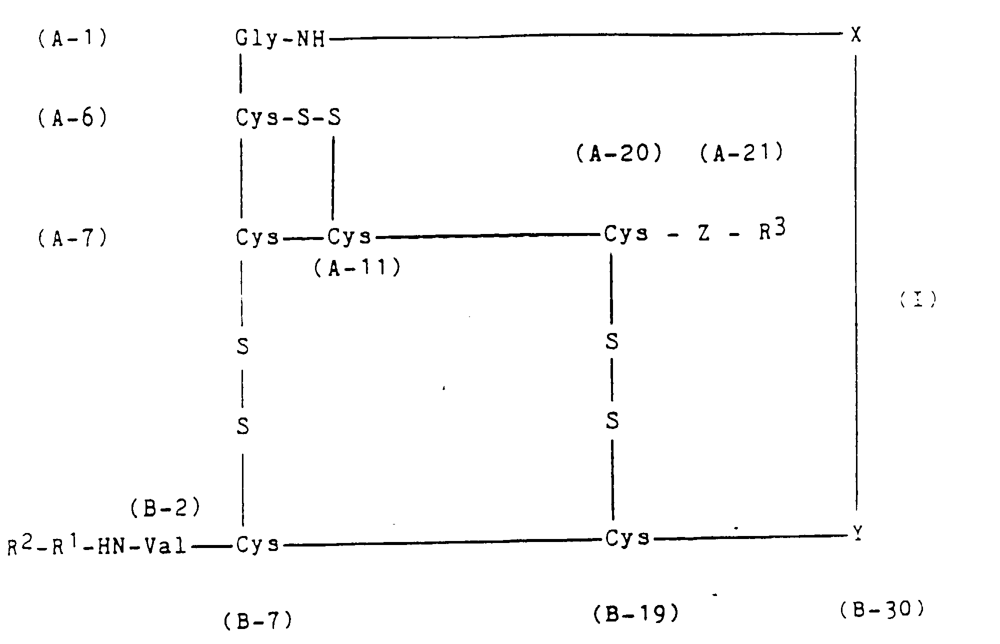
R¹ représente n acides aminés, et n est le nombre entier 0 ou 1,
R² représente l'atome d'hydrogène,
un acide aminé qui peut être éliminé par voie chimique ou enzymatique ou un peptide avec de 2 à 30 résidus d'acides aminés,
R³ représente un groupe hydroxy,
un acide aminé ou
un peptide avec de 2 à 10 acides aminés,
X représente la L-arginine ou
un peptide avec de 2 à 45 acides aminés, sur lequel se trouve un résidu L-arginine à l'extrémité Cet N-terminale,
Y un acide aminé génétiquement codable,
Z un acide aminé génétiquement codable,
A1 à A20 et B2 à B29 représentent une séquence d'acides aminés naturelle d'insulines humaines ou animales ou une séquence modifiée par échange d'un ou de plusieurs résidus d'acides aminés,
procédé caractérisé en ce que, l'on hydrolyse la préproinsuline en présence de clostripaïne et on la transforme éventuellement en l'insuline correspondante avec la carboxypeptidase B.

2. Procédé selon la revendication 1, caractérisé en ce que, l'on utilise la préproinsuline de Formule I, dans laquelle
R¹ représente Phe,
R² représente l'atome d'hydrogène,
un acide aminé naturel ou un peptide comprenant de 2 à 30 acides aminés naturels, qui se termine avec la L-arginine à l'extrémité C-terminale,
R³ représente un groupe hydroxy,
un acide aminé naturel
ou un peptide comprenant de 2 à 10 acides aminés naturels,
X représente la L-arginine ou
une chaîne C d'une proinsuline humaine ou animale,
Y un acide aminé choisi parmi Thr, Ala ou Ser,
Z un acide aminé choisi parmi Asn, Gln, Asp, Glu, Gly, Ser, Thr, Ala ou Met,
A1 à A20 et B2 à B29 représentent une séquence d'acides aminés d'insulines humaines ou animales.

3. Procédé selon la revendication 1 ou 2, caractérisé en ce que, l'on utilise la préproinsuline de Formule I, dans laquelle
R¹ représente Phe,
R² représente l'atome d'hydrogène,
ou un peptide comprenant de 2 à 30 acides aminés naturels, qui se termine avec la L-arginine à l'extrémité C-terminale,
R³ représente un groupe hydroxy,
un acide aminé naturel
ou un peptide comprenant de 2 à 10 acides aminés naturels,
X représente la L-arginine ou
une chaîne C d'une proinsuline humaine, de porc ou de boeuf,
Y Thr,
Z Asn,
A1 à A20 et B2 à B29 représentent la séquence d'acides aminés d'insuline humaine, de porc ou de boeuf.

4. Procédé selon une ou plusieurs des revendications 1 à 3, caractérisé en ce que, l'on opère à une valeur de pH comprise entre 4 et 12, de préférence entre 6 et 9.

5. Procédé selon une ou plusieurs des revendications 1 à 4, caractérisé en ce que, la concentration des préproinsulines de Formule I est comprise entre 0,01 mg/ml et 100 mg/ml, de préférence entre 0,1 mg/ml et 10 mg/ml.

6. Procédé selon une ou plusieurs des revendications 1 à 5, caractérisé en ce que, le rapport préproinsuline-clostripaïne (mg par unités) est de 1 : 0,01 à 1 : 1000, de préférence de 1 : 0,1 à 1 : 50.

7. Procédé selon une ou plusieurs des revendications 1 à 6, caractérisé en ce que, la température de réaction est comprise entre 0°C et +80°C, de préférence entre +20°C et +40°C.

8. Procédé selon une ou plusieurs des revendications 1 à 7, caractérisé en ce que, la carboxypeptidase B est présente en même temps ou est utilisée après coupure avec la clostripaïne.

9. Procédé selon une ou plusieurs des revendications 1 à 8, caractérisé en ce que, la clostripaïne et/ou la carboxypeptidase B se présentent sous une forme immobilisée.
